# EUROPEAN PATENT APPLICATION

(11) **EP 1 092 777 A1**
(43) Date of publication of application: **18.04.2001**
(21) Application number: 99402552.6
(22) Date of filing: 15.10.1999
(51) Int. Cl.: C12N 15/63, C12N 15/67, C12N 15/10, C12Q 1/68

(54) **Self-cleaving RNA sequences and their use for the control of protein synthesis.**

(71) Applicant: Aventis Pharma S.A., 92160 Antony (FR)
(72) Inventor: Piganeau, Nicolas, 35000 Rennes (FR); Famulok, Michael, 53175 Bonn (DE); Thuillier, Vincent, Piedmont, California 94610 (US)

(57) **Abstract**

This invention relates to the control of protein synthesis from a mRNA sequence (activation or repression) by inserting a sequence into an untranslated region (UTR) of a gene which confers conditional self-cleavage to the transcribed RNA.

### Annex to the application documents - subsequently filed sequences listing

SEQUENCE LISTING

<110> RHONE-POULENC RORER S.A.

<120> SELF CLEAVING RNA SEQUENCES AND THEIR USE FOR THE
      CONTROL OF PROTEIN SYNTHESIS

<130> Ribo

<140> 99402552
<141> 1999-10-15

<160> 4

<170> PatentIn Ver. 2.1

<210> 1
<211> 87
<212> DNA
<213> Artificial Sequence

<220>
<223> Description of Artificial Sequence:PRIMER

<400> 1
cgcgttgtgt ttacgcgtct gatgagtnnn nnnnnnnnnn nnnnnnnnnn nnnnnnnnnn 60
nnnnnnnacg aaactacctc gagacgt                                      87

<210> 2
<211> 24
<212> DNA
<213> Artificial Sequence

<220>
<223> Description of Artificial Sequence:PRIMER

<400> 2
cgcgttgtgt ttacgcgtct gatg                                         24

<210> 3
<211> 66
<212> DNA
<213> Artificial Sequence

<220>
<223> Description of Artificial Sequence:PRIMER

<400> 3
agctggtacc taatacgact cactatagga gctcggtagt cacgcgttgt gtttacgcgt 60
ctgatg 66

<210> 4
<211> 20
<212> DNA
<213> Artificial Sequence

<220>
<223> Description of Artificial Sequence:PRIMER

<400> 4
acgtctcgag gtagtttcgt 20

## Description

### Background of the invention

This invention relates to the control of protein synthesis from a mRNA sequence (activation or repression) by inserting a sequence into an untranslated region (UTR) of a gene which confers conditional self-cleavage to the transcribed RNA.

Synthesis of a protein in vivo is controlled at several levels. The first level is the transcription of the gene to generate a pre-messengerRNA (pre-mRNA). The rate of transcription initiation as well as the transcript elongation are under tight physiological control by the basal transcription machinery. Another level is pre-mRNA splicing into a mature mRNA. Alternative splicing events can occur in tissue-specific manner or under hormonal control, hence the active protein is synthesized in a restricted set of tissue or physiological environment. Other levels of control are the mRNA turn-over and the ability of the mRNA to be translated into protein. Hence drug-control of protein synthesis can theoretically be addressed at each of the aforementioned levels.

A drug can control the expression of a target gene within a genome by altering the rate of synthesis of the RNA. It can be achieved by drugs which bind DNA with high affinity and sequence- specificity and hence compete with the transcription factors necessary for the transcription of the target gene. Such drugs may be triple helix-forming oligonucleotides (C. Helene et al., Ciba Found Symp. (1997) 209: 94-106), peptide nucleic acids (Pooga, M. et al.; Nat Biotechnol. (1998) 16(9):857-61) or pyrrole-imidazole polyamides (Dickinson, L.A. et al.; Proc Natl Acad Sci U S A. (1998); 95(22): 12890- 5). It can also achieved by xenogenic transcription factors which bind specifically to the promoter region of the gene and which are activated upon binding of the inducer molecule (Allgood, V.E. et al.; Annual Reports in Medicinal Chemistry (1997) 32: 231-239). The drugs which activate the xenogenic transcription factors are antibiotics, estrogen analogs or immuno-suppressors.

Decreased protein production can be achieved by rising the turnover of the mRNA. Antisense oligonucleotides anneal with the target RNA which can induce its cleavage by RNaseH, or prevent its translation or induce faster degradation by disturbing the secondary structure of the RNA (Kumar, M. et al.; Microbiol Mol Biol Rev. (1998) 62(4):1415-34). In contrast ribozymes anneal with a target RNA and cleave it (Forster, A.C. & Altman, S.; Science (1990) 249: 783-6). Ribozymes are typically RNA molecules which have enzyme-like catalytic activities usually associated with cleavage, splicing or ligation of nucleic acid sequence. The typical substrates for catalytically active ribozymes are RNA molecules, although ribozymes can catalyze reactions in which DNA molecules serve as substrates. Naturally occuring ribozymes which are active intracellularly work in *cis,* catalyzing only a single turnover reaction, and are usually self-modified during the reaction (Cech, T.R.; Biosci Rep. 1990;10(3):239-61). However, ribozymes can be engineered to act in *trans,* in a truly catalytic manner, with a turnover greater than one and without being self-modified. Two distinct regions can be identified in a ribozyme: the binding region which gives the ribozyme its specificity through hybridization to a specific nucleic acid sequence, and a catalytic region which gives the ribozyme the activity of cleavage, ligation or splicing.

Drug-control of the protein production from a specific target gene within a genome can be addressed by controlling the translation of the mRNA into protein. Control of gene expression which takes advantage of the interaction of a specific ligand-binding RNA (aptamer) and its cognate ligand has been published (Werstuck, G. & Green, M.R.; Science (1998) 282, 296-298). Translation of the mRNA is inhibited by a drug which binds in the vicinity of the 5' cap structure of the mRNA. In this setting, the intracellular presence of the drug is repressing protein production.

The control of gene expression and protein production is advantageous in the field of gene therapy and DNA vaccines. It is intended to prevent the occurrence of adverse side effects and to tune the level of expression within an efficient therapeutic window. The use of the above-mentioned systems for the control of gene expression in gene therapy, however, faces major hurdles. The DNA binding drugs, as well as anti-sense and ribozyme oligonucleotides have a poor bio-availability and are readily degraded by nucleases and proteases within tissues or body fluids. Xenogenic transcription factors may elicit a cytotoxic immune response which will destroy the cells expressing the transgenes and hence undermine the therapy or the vaccination (S. K. Tripathy et al., Nature Medicine (1996) 2, 545-550). Furthermore the drugs which activate the xenogenic transcription factors are antibiotics, estrogen analogs or immuno-suppressors. They are not therapeutics per se for the gene therapy and may have undesired side effects. Therefore it would be advantageous to choose an inducer drug which is at least innocuous, for the therapy considered. An additional drawback is the large cloning space needed in the vectors, in order to accommodate the control sequences and the genes encoding the transcription factors. Therefore it is highly advantageous i) to avoid the use of xenogenic proteins for the control of gene expression and ii) to choose the inducer drug according to the therapeutic objectives.

Control of gene expression and protein production is advantageous in the field of functional genomics, transgenic plants and transgenic animals. The conditional expression of a gene into cell cultures or whole plants or animals and the comparison of the phenotypes with and without gene expression enables one to decipher the function of the gene. The drawbacks of the xenogenic transcription factor-based gene switches are two-fold: i) the expression of the target gene and the translation of the mRNA are no more under control of the endogenous physiological stimuli and ii) the effect of the inducer drug on the physiology of the cells. Therefore it is highly advantageous i) to keep the transgene under the control of its endogenous transcription promoter, as well as maintain the same translation control sequences on the mRNA and ii) to choose an inducer drug which does not alter cell physiology. The invention described hereafter features the advantageous characteristics aforementioned.

### Description of the invention

### Definitions

### Gene:

Any nucleic acid molecule encoding a biological product, i.e., a RNA, protein, polypeptide or peptide. A gene, within the context of the instant invention, therefore includes gDNA, cDNA or synthetic or semi-synthetic DNAs. In particular, genes according to the instant invention can be any nucleic acid encoding a biological product, comprising one or more naturally present or artifically produced untranslated region(s).

### Untranslated region (UTR) of a gene:

A DNA sequence of a gene which is transcribed into RNA but which is not translated into a protein. The UTR may be located before the 5'-end of the coding region (5'-UTR), after the 3'-end of the coding (3'-UTR), or may be an intron inserted within the coding region.

### Untranslated region of a pre-mRNA:

A sequence of the pre-mRNA which is not translated into protein. The UTR may be located before the 5'-end of the coding region (5'-UTR), after the 3'-end of the coding (3'-UTR), or may be an intron inserted within the coding region.

### Ligand:

The ligand can be a nucleic acid molecule, a protein, polysaccharide or sugar, or an organic or inorganic molecule which interacts with the self-cleaving RNA sequence and either inhibits or stimulates self-cleavage.

### Description of the invention

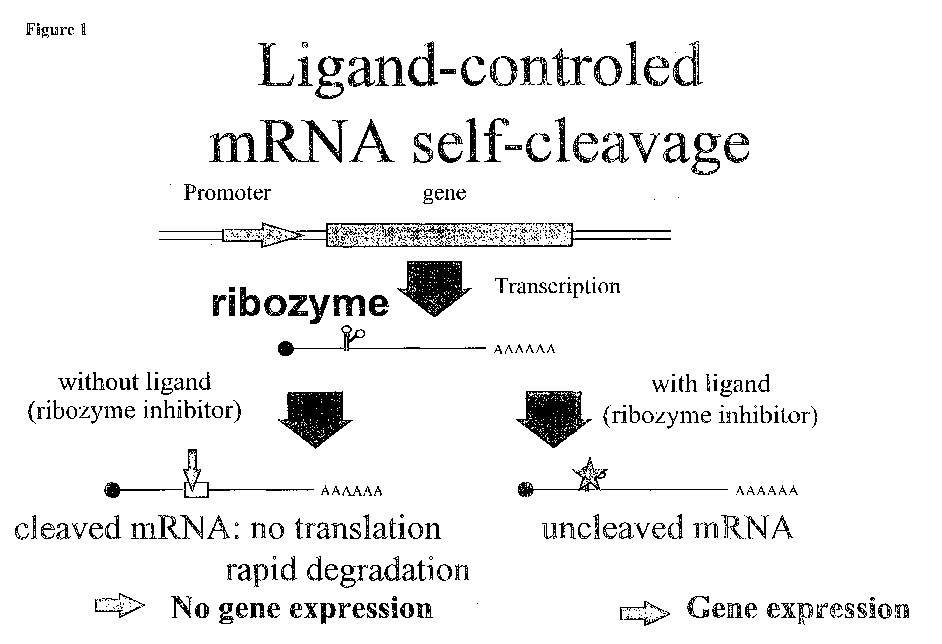

The instant invention provides methods and compositions for the control of protein production in a cell. More particularly, the invention provides methods and compositions for the control of protein production using particular nucleic acid sequences, inserted within at least one UTR of a gene, which confer ligand-dependant self-cleavage to RNAs transcribed from said gene. Hence, the pre-mRNA or the mRNA can undergo self-cleavage that can be regulated through the presence, or the absence of a ligand. The ligand-controlled cleavage enables one to control protein production with this ligand (figure 1).

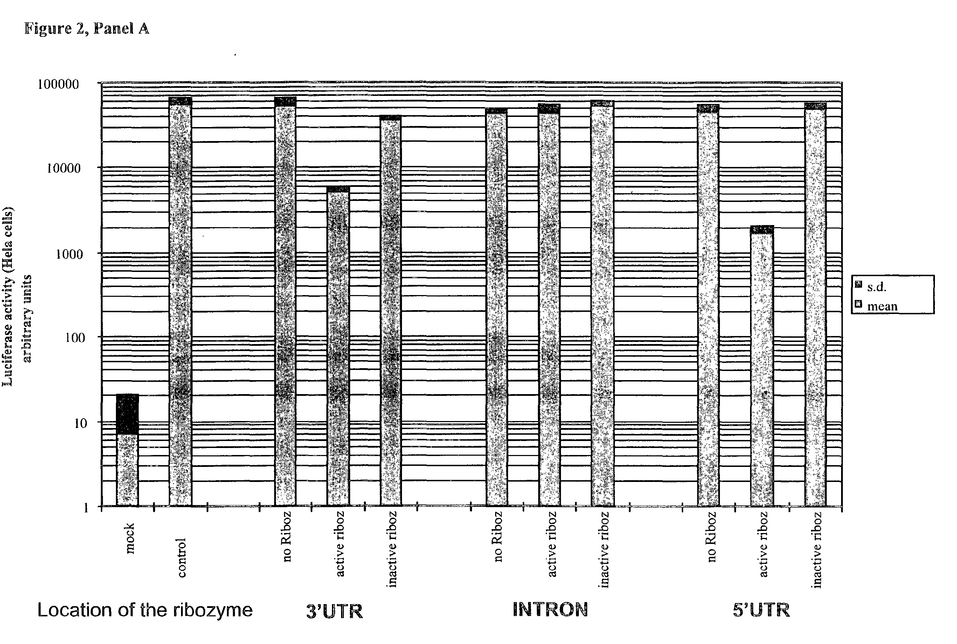

This method is useful in situations where a gene is transfected into cultured cells or into live animals and one needs to control the expression of this gene. The amount of a protein synthesized in a cell is proportional to the amount of its mRNA, and depends on efficient translation of its mRNA into protein. Two features of mRNAs are essential for their efficient translation into proteins (Gallie, R.; Genes & Dev. (1991) 5: 2108-2116) and for their stability (Beelman, C.A. & Parker, R.; Cell (1995) 81: 179-183): the 5'-cap structure and the 3'-polyA tail. Figure 2 shows that the expression of the luciferase gene can be controlled by removing either 3'-polyA or 5'-cap from the mRNA. The insertion of an active nucleic acid sequence of this invention in the 5'- or 3'-UTR of the reporter gene entails the cleavage of the transcribed RNA and hence the removal of, respectively, the 5'-cap and 3'-polyA. The active nucleic acid sequence; in either location; decreases the luciferase protein production whereas an inactive nucleic acid sequence in the same location does not affect gene expression.

This invention can be used to control production of essentially all types of proteins, in essentially all types of cells, in vitro ex vivo or in vivo.
A first object of this invention resides more particularly in a modified gene encoding a protein, polypeptide or peptide, wherein said modified gene contains, inserted in an untranslated region thereof (UTR), a nucleic acid sequence conferring ligand-dependant self-cleavage to a RNA molecule transcribed from the gene.

As indicated above, the modified gene can be a genomic DNA, a cDNA or a synthetic DNA. The modified gene comprises at least one UTR region, which can be naturally present in said gene or artificially inserted therein. For instance, where the gene is a gDNA molecule, the gene comprises naturally-ocurring UTRs such as 5'-UTR, introns and 3'-UTRs. Where the gene is a cDNA or a synthetic (or semi-synthetic) molecule, UTR regions may be introduced, such as introns. Furthermore, even where natural UTRs are present within the gene, additional UTR can be inserted, in addition thereto or in replacement thereof. The modified gene may further comprise a transcriptional promoter, to direct transcription of the coding region into pre-mRNA. The gene may, in addition, be contained in a vector, such as a plasmid, a virus, a cosmid, a phage, an artificial chromosome, etc. The modified gene may encode any type of protein, polypeptide or peptide, including protein, polypeptide or peptide of human, other mammalian, plant, viral or bacterial origin, or derivatives thereof for instance. The encoded biological product may exhibit therapeutic or prophylactic activity, and may also represent a marker molecule, for instance.

As indicated above, the modified gene of this invention comprises at least one particular nucleic acid sequence inserted in a UTR of said gene. The UTR can be a 5'-UTR, a 3'-UTR or an intron. Although the insertion of one single copy of a nucleic acid sequence in a gene of the invention is sufficient to provide control over protein production, in particular embodiments, the modified gene may comprise several copies of such a nucleic acid sequence, inserted in various UTRs or in various locations of a UTR, or different nucleic acid sequences inserted in various UTRs or in various locations of a UTR.

In one embodiment the self-cleavage of the RNA sequence inserted into a UTR of the pre-mRNA is inhibited by a ligand. Hence the pre-mRNA or mRNA is cleaved in the absence of ligand and protein production is decreased ; the pre-mRNA or mRNA is not cleaved in the presence of ligand and protein production is restored.

A particular modified gene of this invention thus comprises a nucleic acid sequence which confers ligand-inhibited self-cleavage to a RNA transcribed from the gene.

In another embodiment the self-cleavage of the RNA sequence inserted into a UTR of the pre-mRNA is activated by a ligand. Hence the pre-mRNA or mRNA is cleaved in the presence of ligand and protein production is repressed; the pre-mRNA or mRNA is not cleaved in the absence of ligand and protein production is restored.

Another particular modified gene of this invention thus comprises a nucleic acid sequence which confers ligand-activated self-cleavage to a RNA transcribed from the gene.

The invention also relates to a method of modifying a gene, comprising inserting, within at least one untranslated region of the gene, a nucleic acid sequence conferring ligand-dependent self-cleavage to a RNA transcribed from said gene.

The nucleic acid can be inserted in various sites within a UTR sequence. Where 5'-UTR or 3'- UTR are concerned, insertion can be performed in different sites which essentially do not affect transcription of the DNA into pre-mRNA. Such sites may be determined by analyzing the sequence of a UTR, and by using restriction sites available or artificially created. Insertion may also be accomplished by recombination, mutagenesis, etc. Where introns are concerned, insertion can take place in any region which do not affect transcription. Since the efficiency of self cleavage of the nucleic acid sequence within a UTR may depend on the surrounding nucleotide sequence, it is preferred to use computer programs like RNAfold (Zuker, M.; Methods Mol Biol (1994) 25:267-94) which predict the most stable conformations, and assess whether the nucleic acid sequence folds in a productive conformation when inserted in a particular location within a UTR. Indeed the surrounding sequence may provide the nucleic acid sequence with alternative folding pathways which lead to inactive or ligand-independent conformations (Stage- Zimmermann, T.K. RNA (1998) 4:875-889). Various sites of insertion of the nucleic acid sequence within a UTR can be selected according to the above methodologies.

The particular nucleic acid sequence used in the instant invention to provide production control can be any nucleic acid sequence encoding a ligand-dependant self-cleavable RNA. The size of this nucleic acid sequence can vary, depending on its nature and/or origin. Generally, the nucleic acid sequence comprises between 10 and 500 base pairs, preferably below 300 base pairs. Typical such nucleic acids comprise between 20 and 200 bp.

The nucleic acid can be of different origin. In particular, the nucleic acid can be derived from naturally occuring self-cleavable RNA sequences. In this regard, one can choose DNA sequences whose RNA transcripts have a self-cleavage activity which is known to be affected by various ligands such as hammerhead ribozymes, hepatitis delta virus ribozymes, Neurospora VS ribozyme, hairpin ribozymes, group I intron, group II intron and Rnase P, for instance (Clouet-d'Orval, B.et al.; Biochemistry (1995) 34: 11186-90; Olive, J.E. et al.; EMBO J. (1995) 14: 3247-51; Rogers, J. et al.; J. Mol. Biol. (1996) 259: 916-25). These sequences can be prepared artificially and used according to the instant invention. In particular, DNAs encoding such RNAs (or functional derivatives thereof) can be prepared by conventional techniques and used to modify a gene in accordance with the instant invention.

Alternatively, any DNA sequences whose RNA transcripts have self-cleaving activity may be chosen and inhibitors of self-cleavage may be identified from a library of compounds.

In a preferred embodiment, however, an advantageous ligand is chosen first and DNA sequences whose RNA transcripts feature ligand-dependant self-cleavage are produced in vitro. In this regard, the invention also describes a particular method that can be used to produce artificial, non-naturally ocurring ligand-dependant self-cleavable RNA and DNA encoding them. Said artificial, non-naturally ocurring ligand-dependant self-cleavable RNA are called aptazymes, and represent another object of the instant invention.

Accordingly, in a particular embodiment of this invention, the nucleic acid sequence is an artificial DNA encoding an aptazyme. Said aptazyme can be obtained by an in vitro evolution and selection method, as described below.

### Aptazyme production

In the method disclosed in this invention, the ligand can be selected first, and then corresponding activated or inhibited aptazymes are produced.

### Selection of the ligand

The ligand can be a nucleic acid molecule, a protein, polysaccharide or sugar, or an organic or inorganic molecule. The nature of the ligand can be chosen to be exogenously supplied, such as some non-toxic molecule or drug which readily enters at least the cells transfected with the transgene. Alternatively, an entirely endogenous system can be designed in which the controlling ligand is a molecule (e.g., some small metabolite or macromolecule) present within the target cell. In this regard, the ligand can be a molecule present within the target cell population and essentially absent (or present at a lower concentration) within other cell population, thereby conferring tissue selectivity to the expression. More particularly, the ligand may be a molecule present within the target cell population, which is directly or indirectly related to a pathology or condition to be corrected, and essentially absent from cells or tissues not affected by the pathology. The activity of the ligand-dependant self-cleavage nucleic acid is then dependent on its binding to the metabolite or macromolecule. If the ligand-dependant self-cleaving nucleic acid is inactivated by (i.e., not cleaved in the presence of) the metabolite or macromolecule (e.g., a pathogenic protein), the expression of the gene is then restricted essentially to the cells which express sufficient amounts of the metabolite or macromolecule, i.e., the diseased cell population (examples of such ligands include mutated p53 molecules, activated oncogenes, etc.). Other examples of ligands include antibiotics (e.g., doxycycline, pefloxacine, etc.), molecules which are used in humans (such as drugs, adjuvents, substitutes, etc.) and any molecule which would be innocuous in a human subject, for instance. This process requires a very high degree of specificity in the molecular recognition between the aptazyme and the ligand. Such a specificity can in principle is achieved by a nucleic acid aptamers (Famulok, M. ; Curr. Opin. Struct. Biol. (1999); 9: 324-329).

### Selection of the aptazyme

Aptazymes that undergo ligand-dependent self-cleavage can be prepared and isolated by an *in vitro* evolution and selection method. This method is related to the SELEX technology (US Patent 5270163, incorporated therein by reference) which is a technique that allows the simultaneous screening of highly diverse combinatorial libraries of different RNA or DNA (single stranded or double stranded DNA) molecules for a particular feature. These features may be i) catalytic activity of a nucleic acid, ii) the ability of a nucleic acid to specifically complex a desired target molecule with high affinity and selectivity.

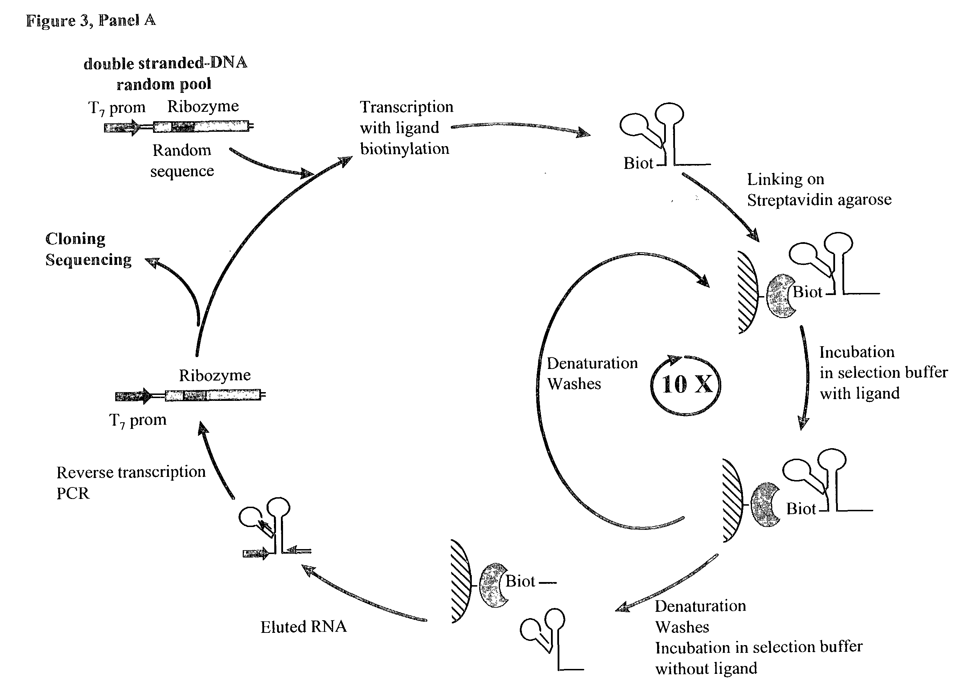

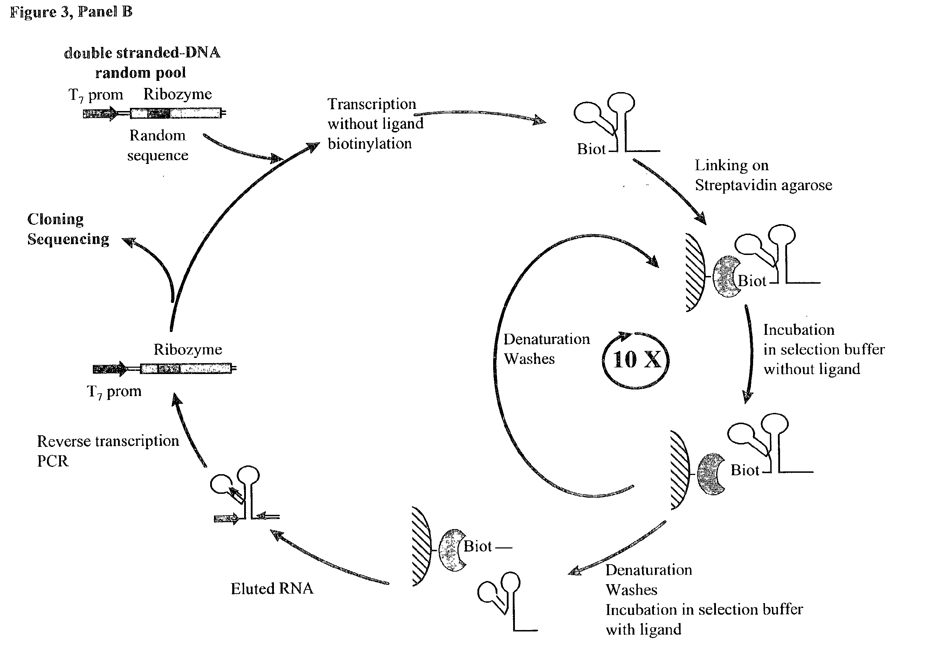

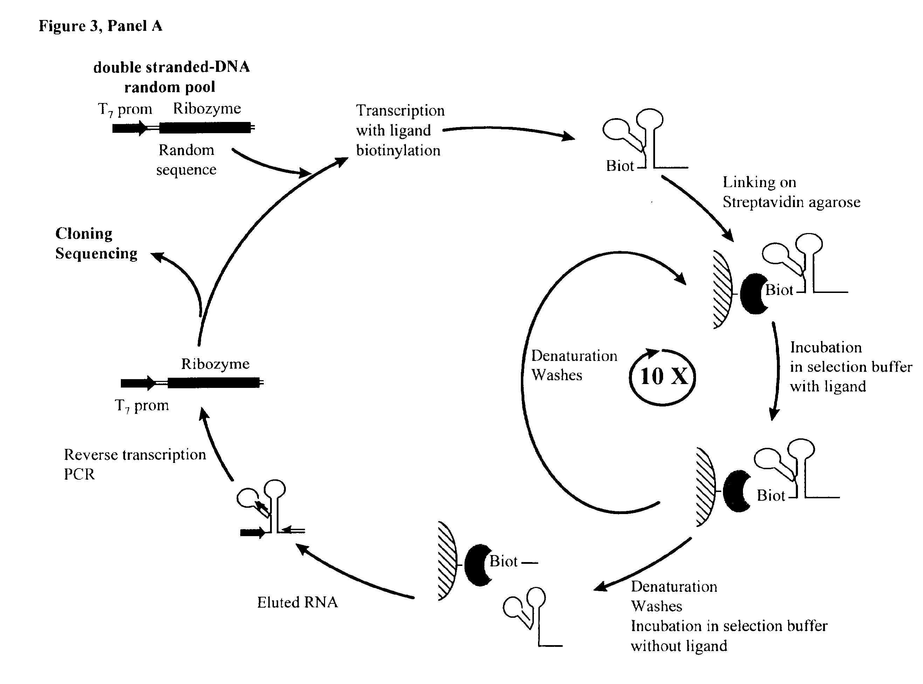

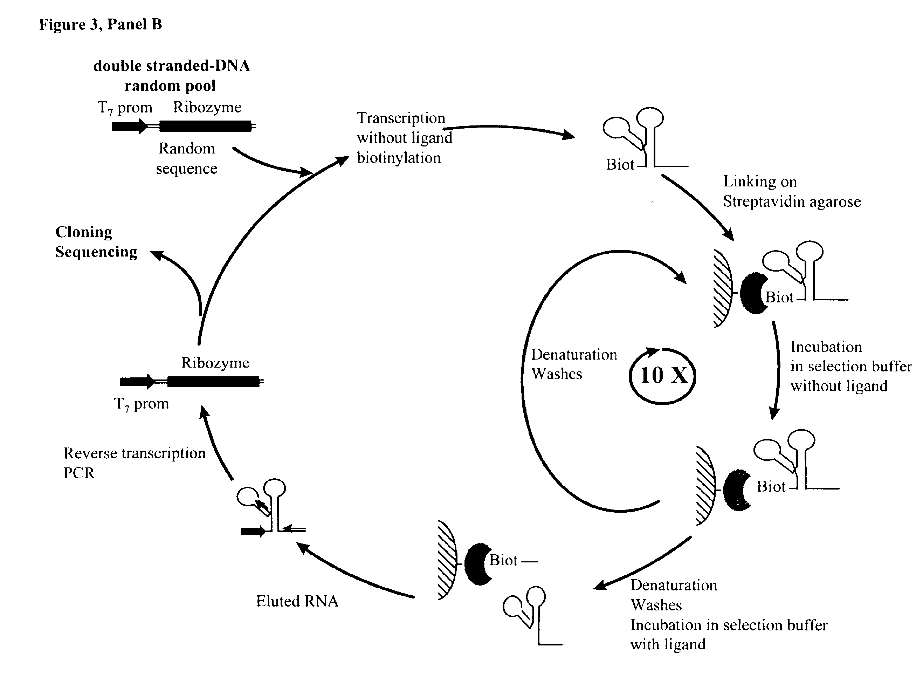

The present method differs from methods which have been previously described for the selection of allosteric ribozymes (WO94/13791; W098/08974). More specifically, a production method of this invention (as depicted in figure 3), comprises:
1. preparing a pool of different double-stranded deoxyribonucleic acid (DNA) molecules, at least part of the sequence in the molecules of the pool is a random or partially random sequence, such that said part has a different sequence in different molecules of the pool. Generally a random sequence may be prepared, for example, by utilizing a nucleic acid synthesizer,
2. transcribing the pool of DNA molecules into a pool of ribonucleic acid (RNA) molecules under conditions where no-self cleavage should occur: in the presence of ligand for the selection of ligand-inhibited self-cleaving nucleic acid sequences, or in the absence of ligand for the selection of ligand-activated self-cleaving nucleic acid sequences,
3. immobilizing all members of the said RNA pool on a solid support under conditions where no self-cleavage should occur (presence or absence of ligand),
4. incubating said RNA pool, in the presence or absence of said ligand, under conditions where no self-cleavage should occur (presence or absence of ligand),
5. separating between the RNA molecules bound to the solid support, and the RNA molecules present in the liquid phase which result from self-cleavage. Denaturing and washing the RNA molecules bound to the solid support,
6. repeating steps 3-5 over a plurality of cycles, e.g. about 1-50 cycles, to eliminate the RNA sequences which are capable of self-cleavage under non-permissive conditions, albeit with low efficiency, due to multiple, non-productive conformational states,
7. adding or removing said ligand to said pool and incubating under conditions permitting self-cleavage, and
8. separating between the RNA molecules of said pool which feature self-cleavage (present in the liquid phase) and such which do not (bound to solid support), to obtain a first selected pool of RNA molecules which feature ligand-dependent self-cleavage.
In a preferred embodiment, the method of this invention further comprises:
9. reverse-transcribing the said first selected pool of RNA molecules into a pool of single-stranded complementary DNA (cDNA) molecules and amplifying (for instance by PCR) the single-stranded cDNA pool into a double-stranded DNA pool, and
10. repeating steps 2-9 over a plurality of cycles, e.g. about 10-100 cycles to obtain said aptazymes.

This method can also be used to select aptamers, i.e. ligand-binding RNA sequences.

In one embodiment, the molecules in the pool are comprised of an entirely random sequence with the exception of two short flanking sequences which encompass the RNA polymerase promoter and the attachment sites of the amplification primers.

In another embodiment of the invention the molecules in the pool are constructed based on a known ribozyme sequence. For example, the molecule may be comprised of a constant, ribozyme-derived sequence attached to a random or semi-random sequence.

There is no set number of random nucleotides. However, in general the random sequence contains between 10 and 300 nucleotides, preferably between 20 and 200, more preferably between 20 and 180 nucleotides. Although referred to herein as a »random» sequence, it is understood that the sequence is random only as originally used in the selection process, that the product of the selection process is not random but a set of specific sequences displaying ligand-dependent self-cleaving ability.

The initial pool may comprise a various number of DNA molecules. In particular, the initial pool may comprise up to 10²⁰ molecules or more. Typical pools comprise between 10⁴ and 10¹⁶ molecules.

It should be understood that the above production method may be applied to different pools of DNA, including genomic, cDNA or RNA libraries, for instance.

In the steps of the selection, it is desired to apply conditions which mimic the cellular environment in which the aptazyme will eventually be used. For example where the aptazyme is intended to control protein production in mammalian cells, the ionic composition of the selection medium will be set accordingly and the temperature will be set to 37 degrees Celsius.

It is useful to progressively increase the threshold criteria for selection in each round of selection and amplification. For example, as the steps of the in vitro evolution proceeds, lower concentration of ligand may be used, resulting in the selection of species having progressively higher affinity to the desired ligand. Number and time of incubation at steps 3-5 (non permissive conditions) may be progressively extended, thus increasing the control of the ligand on the self-cleavage nucleic acid sequences. Time of incubation during step 7, under conditions permitting self cleavage, may be progressively shortened, thus species having faster cleavage rate are selected.

In addition, mutations can be introduced in the selected DNA pool obtained during step 9 by performing the amplification (e.g., the PCR) under mutagenic conditions. This mutagenic step broadens the sequence diversity of the pool which is subjected to the selection.

### Selection of ligand-controlled gene expression cassette

The *in vitro* selection of the aptazyme generally yields a set of several sequences, which exhibit the desired property of ligand-dependent self-cleavage. It is then possible to clone the selected aptazymes and determine their exact sequence. This can be achieved by ligation of the double-stranded DNA pool encoding the aptazymes to an appropriate linearized plasmid vector and transforming bacteria with the resulting circular plasmid vectors (Sambrook et al (1989). Molecular cloning, a laboratory manual, Second Edition, N. Ford, ed., Cold Spring Harbor: Cold Spring Harbor Laboratory Press). The cloned plasmids containing the aptazymes can be sequenced by the method of Sanger *et al.* (Proc. Natl. Acad. Sci. USA, 74 (1977) 5463-5467) with an Applied Biosystems kit according to the manufacturer's instructions. Generally, between 10 and 1000 clones are sequenced, among which a subset of different families exhibiting the desired property can be identified.

It is then desired to select for aptazymes which confer ligand-dependent expression of a reporter gene into the target cells. Reporter genes expression cassettes and vectors carrying them can be constructed by standard molecular biology techniques (Sambrook et al (1989). Molecular cloning, a laboratory manual, Second Edition, N. Ford, ed., Cold Spring Harbor: Cold Spring Harbor Laboratory Press). The target cells are transfected with two reporter genes; reporter gene (1) contains the sequence of an *in* vitro-selected aptazyme within at least one UTR, and reporter gene (2) does not contain any aptazyme. Two sets of cells are cultured in the absence and in the presence of the ligand. The ratio of reporter gene (1) expression over reporter gene (2) expression is assessed in the presence and in the absence of ligand. The aptazyme clone which yields the lowest ratio where protein production is to be repressed and the highest ratio where protein production is to be induced is selected.

The invention can be used to control protein production in vitro, in vivo or ex vivo in various cell types.

In this regard, the invention also resides in a method for controlling production of a protein, polypeptide or peptide from a gene, comprising inserting, within at least one untranslated region of the gene, a nucleic acid sequence conferring ligand-dependent self-cleavage to a RNA transcribed from said gene.

In a particular embodiment, the invention provides a method for removing, in a ligand-dependant manner, the 5'-cap structure and/or the 3'-polyA tail from a pre-mRNA or a mRNA of a gene whose expression is to be controlled. This can be achieved by inserting a sequence in at least one UTR of the gene which entails ligand-dependent self-cleavage of the transcribed RNA.

The invention thus also resides in a method of removing a 5'-cap structure and/or a 3'-polyA tail from a pre-mRNA or a mRNA transcribed from a gene, comprising inserting, within at least one untranslated region of the gene, a nucleic acid sequence conferring ligand-dependent self-cleavage to a RNA transcribed from said gene.

In a particular embodiment, the method is for removing a 5'-cap structure from a pre-mRNA or a mRNA transcribed from a gene, and the nucleic acid sequence is inserted within at least a 5'-untranslated region of the gene.

In another particular embodiment, the method is for removing a 3'-polyA structure from a pre-mRNA or a mRNA transcribed from a gene, and the nucleic acid sequence is inserted within at least a 3'- untranslated region of the gene.

The invention also relates to a method of controlled production of a protein, polypeptide or peptide within a cell, comprising (i) introducing into a cell a modified gene encoding the protein, polypeptide or peptide, wherein said modified gene contains, inserted in an untranslated region thereof (UTR), a nucleic acid sequence conferring ligand-dependant self-cleavage to a RNA molecule transcribed from the gene, and (ii) contacting the cell in the presence or absence of the ligand.

In a particular embodiment, the modified gene contains, inserted in an untranslated region thereof (UTR), a nucleic acid sequence conferring ligand-activated self-cleavage to a RNA molecule transcribed from the gene, and production is repressed by contacting the cell with the ligand, while production is restored (or increased) by removing (or stopping contacting or in the absence of) the ligand.

In another particular embodiment, the modified gene contains, inserted in an untranslated region thereof (UTR), a nucleic acid sequence conferring ligand-inhibited self-cleavage to a RNA molecule transcribed from the gene, and production is increased by contacting the cell with the ligand, while production is depressed (or inhibited) by removing (or stopping contacting or in the absence of) the ligand.

In the above method, the ligand can be either exogenously supplied to the cell or be an endogenous component of the cell, as described above. In this regard, the ligand can be a molecule which is preferentially present in cells where production of the protein, polypeptide or peptide is sought (and essentially absent or express at a lower concentration in other cells or tissues).

The above method can be used for production of a protein, polypeptide or peptide in a cell (or in a cell population or culture) in vitro or ex vivo.

The above method can also be applied to the production of a protein, polypeptide or peptide in a cell, tissue or organ in vivo.

The above cell can be selected from prokaryotic cells, eukaryotic cells, mammalian cells and plant cells, for instance. It may be a tissue, organ, an isolated cell culture, established cell lines or primary cultures. Preferred cells are mammalian cells, in particular murine or human cells, more preferably ex vivo or in vivo. For in vivo uses, the gene can be introduced into the cells by administration to an organism, and the ligand can be supplied exogenously or contained within the cell. Furthermore, the gene may be contained in a vector selected from plasmids, viruses, cosmids, artificial chromosomes, etc., or combinations thereof. Preferred vectors include plasmids and viruses, such as adenoviruses, retroviruses, AAV, HSV, HIV, etc.

The instant invention also discloses and claims combinations of a ligand and a modified gene (or vector containing the same) as described above, for simultaneous, separate or sequential use.

Preferred combinations comprise:
- a ligand and a modified gene, wherein the self-cleavage of the RNA transcribed from the said sequence is inhibited by said ligand;
- a ligand and a modified gene, wherein the self-cleavage of the RNA transcribed from the said gene is activated by said ligand.

Preferably, the ligand is selected from the group of nucleic acid molecules, proteins, polysaccharides, sugars and organic and inorganic molecules.

Other aspects and advantages of the instant invention will be disclosed in the following experimental section, which should be considered as merely illustrative and not limiting the scope of the invention.

### Legend to the Figures

### Figure 1:

Description of the ligand-dependant control of protein production according to the invention

### Figure 2:

Demonstration that a self-cleaving RNA sequence (e.g., a ribozyme hammerhead sequence) inserted within a UTR of a pre-mRNA can control gene expression. Panel A: reporter protein production without ribozyme sequence, with inactive ribozyme sequence or with active ribozyme sequence in one UTR of the gene. Panel B: site of insertion of the ribozyme sequences (restriction enzymes sites) within the pre-mRNA of the reporter gene. Panel C: sequence of the active and the inactive ribozyme.

### Figure 3:

Panel A: outline of the selection procedure for ligand-inhibited self-cleaving RNA sequences.
Panel B: outline of the selection procedure for ligand-activated self-cleaving RNA sequences.

### Figure 4:

Description of the plasmids used for the cloning of the aptazyme pool.

### Figure 5:

Design of the initial pool. The complete sequence of the double stranded DNA molecules used for the first selection cycle is shown, as well as the relative positions and sequences of the different primers synthesized.

### Figure 6:

Time course of the two selections. The percentage of RNA eluted after each selection cycle is shown for the two selections, using doxycycline or pefloxacine as a ligand. An empty space between two cycles indicates an increase in the selection pressure (either by changing incubation times or/and by decreasing ligand concentration (see Table 1).

### Figure 7:

Kinetics of self-cleavage of the selected RNA pool in the presence of increasing amounts of doxycycline. Left panel: pool after selection cycle #10; Right panel pool after selection cycle #13.

### Examples

### Example 1: Selection of aptazymes inhibited by doxycycline and pefloxacine

An *in vitro* selection was carried out to select for aptazymes inhibited by (i.e., not cleaved in the presence of) either doxycycline and pefloxacine (designed as the ligand in sections i to iv). The conditions used during the two selections were identical, except for the ligand used.

### i. Preparation of initial degenerated pool

The initial degenerated pool was produced using standard DNA synthesis chemistry on an Expedite nucleic acids synthesis system (Millipore). Oligonucleotides were purified on denaturating polyacrylamide gels. The following primers were synthesized:

### Pool-Primer:

5’-CGCGTTGTGTTTACGCGTCTGATGAGT-N(40)-ACGAAACTACCTCGAGACGT (SEQ
ID NO: 1)
Primer 1:
5’-CGCGTTGTGTTTACGCGTCTGATG (SEQ ID NO:2)
Primer 2:
5’-AGCTGGTACCTAATACGACTCACTATAGGAGCTCGGTAGTCACGCGTTGTGTTTAC
GCGTCTGATG (SEQ ID NO:3)
Primer 3:
5’-ACGTCTCGAGGTAGTTTCGT (SEQ ID NO:4)

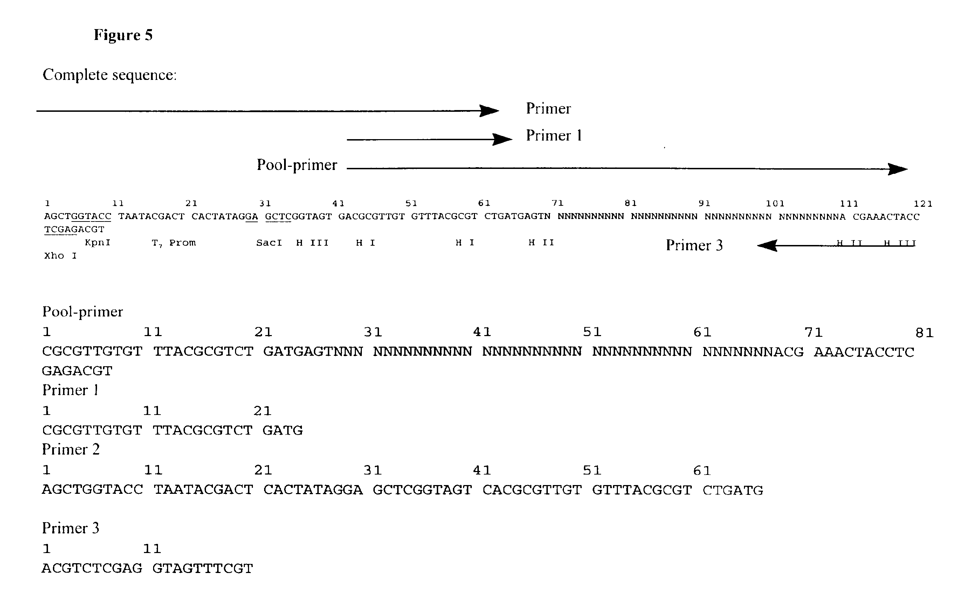

After purification, 6 nmol of Pool-Primer were obtained (4.10¹⁵ molecules). The total DNA was amplified by PCR using Primer 1 and 3 as previously described (Bartel D.P. & Szostak J.W.; Science (1993) 261: 1411-1418). A further amplification step was performed under the same conditions using 6 nmol of pre-amplified double stranded product as template, primer 2 and 3. The final DNA molecules contained the following features (from 5' to 3'): KpnI restriction site, T₇ RNA polymerase promoter, SacI restriction site, Hammerhead ribozyme sequence containing 40 random nucleotides located in the Helix II domain, Xhol restriction (see Figure 5).

This DNA library was used as a template for T₇ RNA polymerase during the initial *in vitro* selection cycle.

### ii. General selection scheme

The selection scheme used is outlined in Figure 3 - Panel A. Several modifications of the selection scheme were done during the selection and are detailed later (section iii).

### a) Transcription and immobilization on a column

Standard transcription reactions contained the following components: 250 units T₇ RNA polymerase (Stratagene), T₇ Buffer (Stratagene), 2,5 mM of each dNTP, 20 mM of GMPS, 20 µCi α³²P GTP, RNasin (50 U), ligand (lmM). The transcription reaction was incubated over night at 37°C. The uncleaved RNA transcripts were then purified on a 8% denaturating polyacrylamide gel. After purification, the RNA was treated with Iodo-Acetyl-LC-Biotin (Pierce - 200 fold excess) for 90 min. at room temperature. Primed RNA was gel purified under the same conditions as described above and incubated with streptavidine agarose (Pierce) for 30 min. at room temperature. The column material was then washed 6 times alternatively in lml washing buffer A (WA ; 25 mM HEPES pH 7.4, 1M NaCl, 5mM EDTA), washing buffer B (WB ; 3M urea, 5mM EDTA) and water. Finally, it was rinsed five times with water.

### b) Negative selection

The immobilized RNA was incubated at 37°C for 2 to 4 hours in selection buffer (SB ; 40 mM Tris HCl pH 8, 50 mM NaCl, 10 mM Spermidine, 8 mM MgCl₂;) in the presence of the ligand (1 mM to 1 µM). The incubation was started upon the addition of magnesium. After round 5, this incubation was interrupted 10 times during the first 2h30 of incubation and the column was washed twice with 1 ml WB (denaturating conditions) and rinsed 3 times with water. The column material was then washed again thoroughly (6 times 1 ml WA - WB - H₂O; 5 times 1 ml H₂O).

### c) Positive selection

The positive selection was performed under similar conditions as the negative selection, without the ligand. Incubation time was considerably shorter (10 to 1 min.). Cleaved nucleic acid sequences were eluted with 1 ml of WB. Eluted RNA was purified by 2 phenol-chloroform extractions and ethanol precipitation.

### d) Reverse transcription and amplification

The RNA was finally reverse transcribed with Tth DNA polymerase (Boehringer) and amplified by PCR using primers 3 and 4 under standard conditions. This DNA template was used as a template for T₇ RNA polymerase for the next round of selection.

### iii. Modifications of the selection scheme during selection

During selection, several modifications of the selection scheme were made (see Table 1). During the first cycle, 16 nmol of transcribed RNA were used in order to preserve the diversity of the pool. Column material and washing volumes were scaled up accordingly . During later cycles, RNA quantity was reduced. The ligand concentration, and the different incubation times were also adjusted to increase the stringency. After selection cycles 10, and 13, the pool was subjected to a mutagenic PCR under previously described conditions (Cadwell R.C. & Joyce G.F.; in PCR Methods and Applications (1992): 28-33). The mutagenic PCR creates a new diversity of RNA molecules closely related to selected sequences.

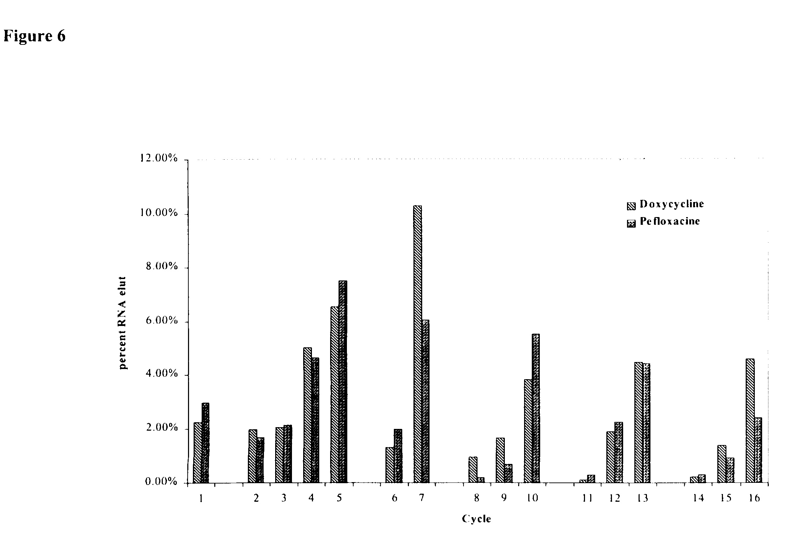

### iv. Enrichment during selection (Figure 6)

Elution profiles during selections for doxycycline inhibited aptazymes and pefloxacine inhibited aptazymes were extremely similar, suggesting that this type of selection scheme is extremely robust. First hints for enrichment could be observed during cycle 4 and were confirmed during cycle 5. However a time course experiment of the selected pools showed no significant difference between cleavage in the presence or absence of the ligand. Furthermore, cleavage efficiency of this pool was severely reduced compared to the unselected pool (data not shown). It was hypothesized that this result was due to coselection of RNA species able of self-cleavage under non-permissive conditions, albeit, with low efficiency, due to multiple non productive conformational states.

To counter-select against these parasites, the selection scheme was modified by introduction of several denaturation steps during the negative selection (see ii-b).

During cycle 7 a new enrichment could be observed. Thus, selection stringency was increased by reducing ligand concentration to 100 µM and elution time to 1 min. After 3 cycles a new enrichment could be observed. The selected pool at this stage was subjected to mutagenic PCR and the selection stringency was increased by a 10 fold decrease in ligand concentration. After 3 cycles, a new enrichment could be observed. This procedure (mutagenic PCR - increase in selection stringency - 3 selection cycles) was reproduced on the selected pool and yielded a new enrichment of the pool. After this stage, a last cycle was performed with a highly reduced elution time (10 sec.), to select for the fastest cleaving nucleic acid sequences.

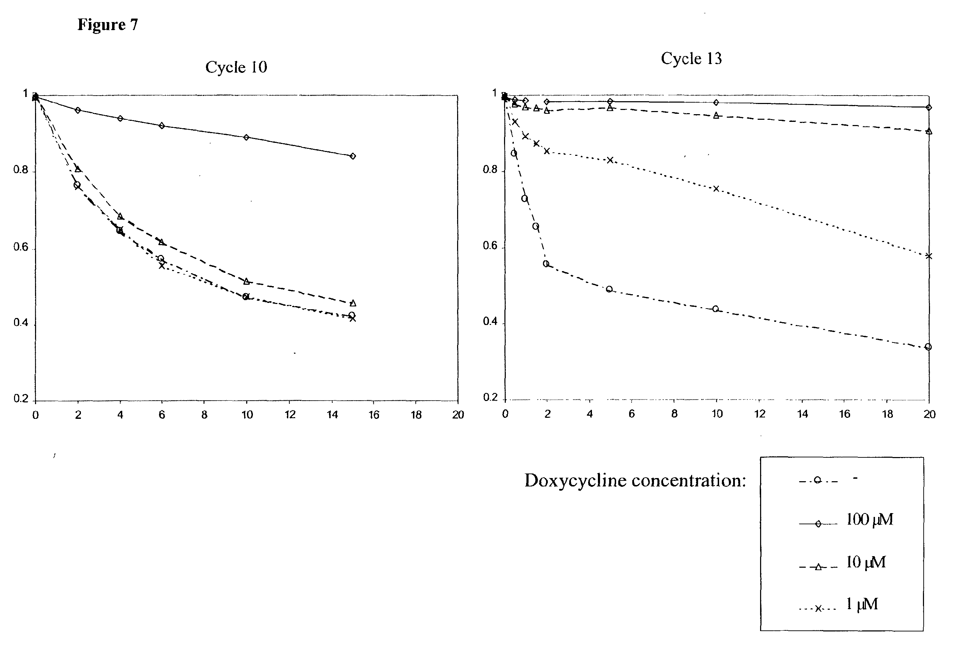

### v. Pool kinetics (figure 7)

During and after selection, pools obtained after different cycles were transcribed under standard conditions (250 units T₇ RNA polymerase (Stratagene), T₇ Buffer (Stratagene), 2,5 mM of each dNTP, 20 µCi α³²P GTP, 50 U RNasin , 1mM ligand). Uncleaved transcripts were gel purified, dephosphorylated using Calf Intestinal Alkaline Phosphatase (Stratagene) and 5' radio-labeled using T₄ polynucleotide kinase (Stratagene) and γ³²P-ATP. Radio-labeled RNA molecules were gel purified and incubated at a 10 nM concentration in SB with various amounts of ligand at 37°C. Reaction was initiated upon addition of magnesium. Aliquots were taken at different time points and mixed with an equal volume of loading buffer (9M urea, 5 mM EDTA) on ice to quench the reaction. The samples were then loaded on a 12 % sequencing gel, and the bands corresponding to intact (103 nt) or cleaved (13 nt) nucleic acid sequences were quantified using a phosphor imager (Molecular Dynamics).

### Example 2: Selection of doxycycline- and pefloxacine-induced expression cassettes in mammalian cells

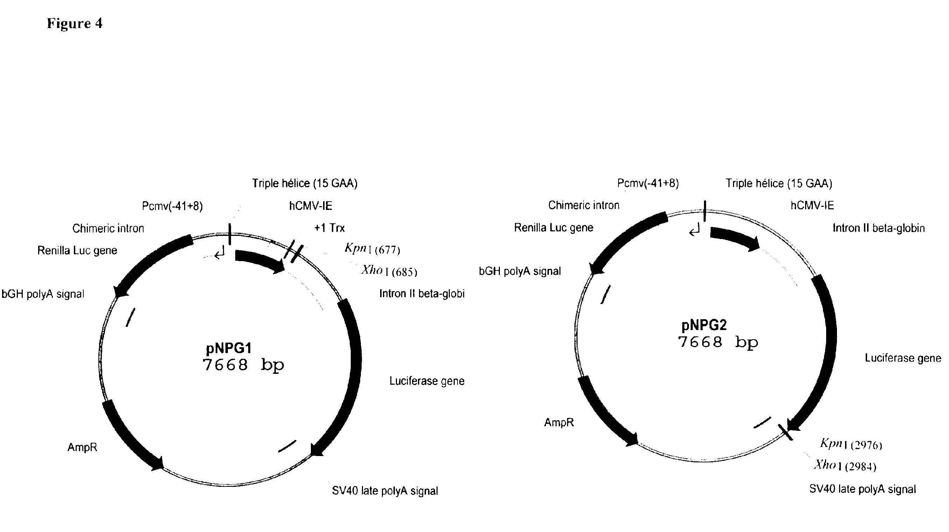

The DNA pools obtained after selection cycles 10, 13 and 16 are cut by the restriction enzymes Xhol and KpnI. The plasmids pNPG1 and pNPG2 (figure 4) are cut with the same enzymes and the three DNA pools are ligated to each of the two plasmids. E. Coli strain DH5a is transfected by each one of the six ligation mixture according to standard procedures (Sambrook et al (1989). Molecular cloning, a laboratory manual, Second Edition, N. Ford, ed., Cold Spring Harbor: Cold Spring Harbor Laboratory Press). One hundred colonies are isolated for each transfection. Plasmid DNA is extracted and purified from each of these colonies. The aptazymes cloned into the plasmids are sequenced by the method of Sanger *et al.* (Proc. Natl. Acad. Sci. USA, 74 (1977) 5463-5467) with an Applied Biosystems kit according to the manufacturer's instructions.

Human cultured cell lines HeLa and HEK293 are transfected by plasmid DNA complexed to lipofectamine (Gibco-Life Sciences, Bethesda, MD USA) according to the manufacturer's instructions (6 ml lipofectamine per mg DNA). In one set of experiments the cells are incubated with 10 mM doycycline or 10 mM pefloxacine, in another set of experiment the cells are incubated without ligand. 48 hours after transfection, the cells are lysed and the amount of Renilla luciferase and firefly lucifease are recorded according to the manufacturer's instructions (Promega, Madison, WI USA). The ratio of firefly luciferase activity (aptazme-controlled) over Renilla luciferase activity (Ratio R) is computed for each experimental condition (i.e. plasmid, ligand). The plasmids which yield production of high amount of firefly luciferase in the presence of ligand (doxycycline or pefloxacine) and low amount of firefly luciferase in the absence of ligand are identified by the highest ratio R. These plasmids are selected for further use in gene transfer experiments where production of a protein is to be controlled by doxycycline or pefloxacine.

### Example 3: Doxycycline- induced protein production in mice

10 mg of aptazyme-containing plasmids pNPG1 or pNPG2 (selected as in example 2) are injected into tibialis cranialis muscles (posterior limbs) of eight weeks-old Balb/C mice, the limbs are subsequently subjected to electroporation in order to enhance gene transfer (Mir, L.M. et al.; P.N.A.S. USA (1999); 96(8):4262-7). One group of five mice is given doxycycline in the drinking water (0.2 mg/ml), the other group is not. 7 days after gene transfer the animals are sacrificed and the tibialis cranialis muscles are collected. The muscles are resuspended in lml of lysis buffer (Dual-Luciferase reporter Assay System, Promega, Madison, WI USA) homogenized by glass and ceramic beads into a Fast-Prep homogenizer (Bio 101, Vista, CA USA). The amount of firefly luciferase and Renilla luciferase are assessed according to Promega's instructions. The amount of firefly luciferase synthesized by the mice of each group (treated or not by doxycycline) is compared. Inter-individual variations in gene transfer efficiency can be compensated by comparing the ratio firefly luciferase over renilla luciferase between the 2 groups of mice. Hence one can verify that luciferase production in mice is induced by doxycycline.

**Table 1**

| Cycle | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| RNA amount | 16 nmol | 1 nmol | | | | | | | | 500 pmol | | | | 100 pmol | | | |
| Ligand Concentration | 1 mM | | | | | | | 100 µM | | | 10 µM | | | 1 µM | | | |
| Negative selection incubation time | 2 hr. | 4 hr. | | | | 10X 15 min. + 90 min. | | | | | | | | | | | |
| Positive selection incubation time | 10 min. | 5 min. | | | | | | 1 min. | | | | | | | | | |
| Mutagenic PCR | * * | | | | | | | | | | | | | | | | |

## Claims

1. A modified gene encoding a RNA, protein, polypeptide or peptide wherein said modified gene contains, inserted in an untranslated region thereof (UTR), a nucleic acid sequence conferring ligand-dependant self-cleavage to a RNA molecule transcribed from the gene.

2. A modified gene of claim 1, wherein said gene is a genomic DNA.

3. A modified gene of claim 1, wherein said gene is a cDNA.

4. A modified gene of claim 1, wherein said gene is a synthetic DNA comprising at least one untranslated region.

5. A modified gene of claim 1, wherein the untranslated region is selected from 5'-UTR, 3'- UTR and introns.

6. A modified gene of claim 1, wherein the untranslated region is naturally present in the gene or has been introduced into said gene.

7. A modified gene of claim 1, wherein said nucleic acid sequence confers ligand-activated self-cleavage to a RNA transcribed from the gene.

8. A modified gene of claim 1, wherein said nucleic acid sequence confers ligand-inhibited self-cleavage to a RNA transcribed from the gene.

9. A modified gene of claim 1, wherein said nucleic acid sequence is derived from hammerhead ribozymes, hepatitis delta virus ribozymes, Neurospora VS RNA, hairpin ribozymes, group I intron, group II intron and Rnase P.

10. A modified gene of claim 1, wherein said nucleic acid sequence is an artificial DNA encoding an aptazyme, obtainable by in vitro evolution and selection.

11. A modified gene of claim 1, wherein the ligand is selected from nucleic acids, proteins, polysaccharides, sugars, organic and inorganic molecules.

12. A modified gene of claim 1, further comprising a transcriptional promoter.

13. A vector comprising a modified gene of claim 1 or 12.

14. A method for controlling production of a protein, polypeptide or peptide from a gene, comprising inserting, within at least one untranslated region of the gene, a nucleic acid sequence conferring ligand-dependent self-cleavage to a RNA transcribed from said gene.

15. A method of modifying a gene, comprising inserting, within at least one untranslated region of the gene, a nucleic acid sequence conferring ligand-dependent self-cleavage to a RNA transcribed from said gene.

16. A method of removing a 5'-cap structure and/or a 3'-polyA tail from a pre-mRNA or a mRNA transcribed from a gene, comprising inserting, within at least one untranslated region of the gene, a nucleic acid sequence conferring ligand-dependent self-cleavage of a RNA transcribed from said gene.

17. A method of claim 16, for removing a 5'-cap structure from a pre-mRNA or a mRNA transcribed from a gene, comprising inserting, within at least a 5'-untranslated region of the gene, a nucleic acid sequence conferring ligand-dependent self-cleavage of a RNA transcribed from said gene.

18. A method of claim 16, for removing a 3'-polyA tail structure from a pre-mRNA or a mRNA transcribed from a gene, comprising inserting, within at least a 3'-untranslated region of the gene, a nucleic acid sequence conferring ligand-dependent self-cleavage of a RNA transcribed from said gene.

19. A method of controlled production of a protein, polypeptide or peptide within a cell, comprising (i) introducing into a cell a modified gene encoding the protein, polypeptide or peptide, wherein said modified gene contains, inserted in an untranslated region thereof (UTR), a nucleic acid sequence conferring ligand-dependant self-cleavage to a RNA molecule transcribed from the gene, and (ii) contacting the cell in the presence or absence of the ligand.

20. A method of controlled production of a protein, polypeptide or peptide within a cell, comprising (i) introducing into a cell a modified gene encoding the protein, polypeptide or peptide, wherein said modified gene contains, inserted in an untranslated region thereof (UTR), a nucleic acid sequence conferring ligand-activated self-cleavage to a RNA molecule transcribed from the gene, and (ii) contacting the cell with the ligand to repress production or in the absence of the ligand to increase production.

21. A method of controlled production of a protein, polypeptide or peptide within a cell, comprising (i) introducing into a cell a modified gene encoding the protein, polypeptide or peptide, wherein said modified gene contains, inserted in an untranslated region thereof (UTR), a nucleic acid sequence conferring ligand-inhibited self-cleavage to a RNA molecule transcribed from the gene, and (ii) contacting the cell with the ligand to increase production or in the absence of the ligand to decrease production.

22. A method of claim 19, 20 or 21, wherein the ligand is exogenously supplied to the cell.

23. A method of claim 19, 20 or 21, wherein the ligand is an endogenous component of the cell, either pathogenic or non pathogenic.

24. A method of claim 23, wherein the ligand is a molecule which is preferentially present in cells where production of the protein, polypeptide or peptide is sought.

25. A method of claim 24, wherein the ligand is a molecule which is present in target cells where production of the protein, polypeptide or peptide is sought and essentially absent or present at lower levels in other cells.

26. A method of claims 19, 20 or 21, for production of a protein, polypeptide or peptide in a cell in vitro or ex vivo.

27. A method of claims 19, 20 or 21, for production of a protein, polypeptide or peptide in a cell, tissue or organ in vivo.

28. Method of claim 19, 20 or 21, wherein the cell is a selected from prokaryotic cells, eukaryotic cells, mammalian cells and plant cells.

29. Method of claim 19, 20 or 21, wherein the gene is contained in a vector selected from plasmids, viruses or combinations thereof.

30. A combination of ligand and modified gene of claim 1, for simultaneous, separate or sequential use.

31. A combination of a ligand and a modified gene according to claim 30, wherein the self-cleavage of the RNA transcribed from the said sequence is inhibited by said ligand.

32. A combination of ligand and a modified gene according to claim 30, wherein the self-cleavage of the RNA transcribed from the said gene is activated by said ligand.

33. A combination according to claim 30, 31 or 32, wherein the ligand is selected from the group of nucleic acid molecules, proteins, polysaccharides, sugars and organic and inorganic molecules.

34. An *in vitro* evolution and selection method for the production of a ligand-dependent self-cleaving RNA sequence, the method comprising:
a) preparing a pool of different double-stranded deoxyribonucleic acid (DNA) molecules, at least part of the sequence in the molecules of the pool is a random or partially random sequence, such that said part has a different sequence in different molecules of the pool,
b) transcribing the pool of DNA molecules into a pool of ribonucleic acid (RNA) molecules under conditions where no-self cleavage should occur: in the presence of ligand for the selection of ligand-inhibited self-cleaving nucleic acid sequence, or in the absence of ligand for the selection of ligand-activated self-cleaving nucleic acid sequence,
c) immobilizing all members of the said RNA pool on a solid support under conditions where no self-cleavage should occur (presence or absence of ligand),
d) incubating said RNA pool, in the presence or absence of said ligand, under conditions where no self-cleavage should occur,
e) separating between the RNA molecules bound to the solid support, and the RNA molecules present in the liquid phase which result from self-cleavage, and denaturing and washing the RNA molecules bound to the solid support,
f) repeating steps d)-e) over a plurality of cycles, e.g. about 1-50 cycles, to eliminate the RNA sequences which are capable of self-cleavage under non-permissive conditions, albeit with low efficiency, due to multiple, non-productive conformational states.
g) adding or removing said ligand to said pool and incubating under conditions permitting self-cleavage, and
h) separating between the RNA molecules of said pool which feature self-cleavage (present in the liquid phase) and such which do not (bound to solid support), to obtain a first selected pool of RNA molecules which feature.

35. The method of claim 34, further comprising:
i) reverse-transcribing the first selected pool of RNA molecules of step h) into a pool of single-stranded complementary DNA (cDNA) molecules and amplifying the single-stranded cDNA pool into a double-stranded DNA pool,
j) repeating steps b)-i) over a plurality of cycles, e.g. about 10-100 cycles to obtain double-stranded DNA molecules encoding ligand-dependent self-cleavage RNA molecules.

36. The method of claim 34 or 35, wherein the molecules of the pool of DNA molecules in step a) have a sequence which is derived from a known self-cleaving RNA sequence with some proportion of the nucleotides being replaced by 10 to 100 random nucleotides.

37. The method of claim 34 or 35, wherein the molecules of the pool of DNA molecules in step a) have a sequence which is derived from a known self-cleaving RNA sequence with some proportion of the nucleotides being replaced by the sequence of a known RNA ligand (aptamer).

38. The method of claim 34 or 35, wherein the molecules of the pool of DNA molecules in step a) have a sequence which is comprised of an entirely random sequence with the exception of two short flanking sequences which encompass the RNA polymerase promoter and the attachment sites of the amplification primers.

39. A synthetic, non-naturally occuring DNA molecule encoding a ligand-dependant self-cleavable RNA sequence.

40. The DNA of claim 39, wherein said ligand is a nucleic acid molecule, a protein, polysaccharide or sugar, or an organic or inorganic molecule.

41. The DNA of claim 39, wherein said ligand is an endogenous component of the cell.

42. A synthetic, non-naturally occuring ligand-dependant self-cleavable RNA molecule.

43. An *in vitro* evolution and selection method for the production of a ligand-binding RNA sequence, the method comprising:
a) preparing a pool of different double-stranded deoxyribonucleic acid (DNA) molecules, at least part of the sequence in the molecules of the pool is a random or partially random sequence, such that said part has a different sequence in different molecules of the pool,
b) transcribing the pool of DNA molecules into a pool of ribonucleic acid (RNA) molecules under conditions where no-self cleavage should occur: in the presence of ligand for the selection of ligand-inhibited self-cleaving nucleic acid sequence, or in the absence of ligand for the selection of ligand-activated self-cleaving nucleic acid sequence,
c) immobilizing all members of the said RNA pool on a solid support under conditions where no self-cleavage should occur (presence or absence of ligand),
d) incubating said RNA pool, in the presence or absence of said ligand, under conditions where no self-cleavage should occur,
e) separating between the RNA molecules bound to the solid support, and the RNA molecules present in the liquid phase which result from self-cleavage, and denaturing and washing the RNA molecules bound to the solid support,
f) repeating steps d)-e) over a plurality of cycles, e.g. about 1-50 cycles, to eliminate the RNA sequences which are capable of self-cleavage under non-permissive conditions, albeit with low efficiency, due to multiple, non-productive conformational states.
g) adding or removing said ligand to said pool and incubating under conditions permitting self-cleavage, and
h) separating between the RNA molecules of said pool which feature self-cleavage (present in the liquid phase) and such which do not (bound to solid support), to obtain a first selected pool of RNA molecules which feature.
